# EUROPEAN PATENT APPLICATION

(11) **EP 4 666 995 A1**
(43) Date of publication of application: **24.12.2025**
(21) Application number: 25183031.1
(22) Date of filing: 16.06.2025
(51) Int. Cl.: A61K 8/44, A61K 8/73, A61K 31/198, A61K 31/728, A61Q 19/08

(54) **ADDITIVE WITH ANTI-IALURONIDASE ACTIVITY AND ITS USE IN HYALURONIC ACID FORMULATIONS**

(30) Priority: 19.06.2024 IT 202400014059
(71) Applicant: Professional Dietetics S.p.A., 20129 Milano (MI) (IT)
(72) Inventor: GIORGETTI, Paolo, 20129 MILANO (IT)
(74) Representative: Bianchetti & Minoja with Trevisan & Cuonzo IPS SRL

(57) **Abstract**

Disclosed is an additive with anti-hyaluronidase activity comprising at least one positively charged amino acid and at least one hydrophobic amino acid, its use for counteracting the degradation of fillers or topical formulations based on hyaluronic acid and a method for inhibiting the degradation of injected hyaluronic acid for cosmetic purposes.

## Description

The present invention relates to an additive with anti-hyaluronidase activity and its use in injectable or topical formulations of hyaluronic acid.

### Background of the invention

Hyaluronic acid (HA) is a high molecular mass glycosaminoglycan composed of repeating disaccharide units of D-glucuronic acid (GlcUA) and N-acetyl-D-glucosamine (GlcNAc). HA is strongly involved in critical biological processes such as cell motility, proliferation, differentiation, and migration. The enzymes that degrade HA, hyaluronidases (HAases), are commonly classified into three groups based on their HA degradation mechanism: hyaluronate 4-glycanhydrolase (hyaluronoglucosaminidase: EC 3.2.1.35), hyaluronate 3-glycanhydrolase: hyaluronoglucuronidase: EC 3.2.1.36) and hyaluronate lyase (EC 4.2.2.1) [1].

Thus, the term hyaluronidase has been introduced to denote the hyaluronic acid (HA) degrading enzymes, which have critical applications in many biotechnological and therapeutic processes. HAases present in various mammalian tissues, belonging to the first group, are of particular interest since they have been shown to be involved in the pathophysiology of many pathologies such as cancer and rheumatoid arthritis [2].

HA has been used in various forms, such as hydrogels, dermal fillers, intradermal injection, scaffolds, creams, films, foams and gels to treat various diseases. HA has demonstrated a wide range of pharmacological activities, including anti-inflammatory [3], wound healing and tissue regeneration [4], immunomodulatory [5], anticancer and antiproliferative [6], antidiabetic [7], anti-aging [8], skin repairing [9] and cosmetic [10] ones. HA plays a multifaceted role in the regulation of various biological processes and in the maintenance of homeostasis in the body. HA is characterized by high hydrophilicity [11, 12] and overall negative electrical charge [13].

Many researchers have investigated the cosmetic and nutricosmetic efficacy of HA-based formulations for skin rejuvenation. An evidence-based meta-analysis revealed that HA exhibits remarkable cosmetic and nutricosmetic efficacy in correcting various skin defects, such as wrinkles, nasolabial folds and skin aging. To study the cosmetic effects, HA has been used in different forms (gels, creams, intra-dermal injections, dermal fillers, facial fillers, autologous fat gels, lotions, serums and implants, etc.).

HA-based compositions, both injectable and topical, suffer from the problem of HA degradation by hyaluronidases. Several solutions have been proposed to overcome this problem: among these, the use of collagen hydrolysates (US 10279076), chondroitin sulphate, calcium hydroxyapatite used as a crosslinker of hyaluronic acid.

However, there remains a need for safe and economical additives that, by inhibiting the activity of hyaluronidases, can preserve hyaluronic acid from degradation.

### DESCRIPTION OF THE INVENTION

It has now been found that a mixture of amino acids comprising at least one positively charged amino acid and at least one hydrophobic amino acid inhibits hyaluronidases in a dose-dependent manner.

In a first aspect, the invention therefore relates to an additive with anti-hyaluronidase activity comprising a mixture of glycine, alanine, valine, leucine, proline and lysine.

The amino acids used according to the invention may be of the L- or D- series or mixtures thereof. Amino acids of the natural L series are preferred, more preferably in the following weight ratios:
- Glycine 1;
- L-Proline: 0.7-0.8;
- L-Alanine: 0.47-0.76;
- L-Valine: 0.35-0.56;
- L-Leucine: 0.13-0.27;
- L-Lysine hydrochloride: 0.10-0.12

Preferred weight ratios are:
- Glycine 1;
- L-Proline: 0.75;
- L-Alanine: 0.48-0.51;
- L-Valine: 0.35-0.37;
- L-Leucine: 0.13-0.15;
- L-Lysine hydrochloride: 0.10-0.11,
or:
- Glycine 1;
- L-Proline: 0.75;
- L-Alanine: 0.75-0.76;
- L-Valine: 0.54-0.56;
- L-Leucine: 0.13-0.14;
- L-Lysine hydrochloride: 0.10-0.11,
or
- Glycine 1;
- L-Proline: 0.75;
- L-Alanine: 0.49-0.51;
- L-Valine: 0.35-0.36;
- L-Leucine: 0.26-0.27;
- L-Lysine hydrochloride: 0.10-0.11.

The additive of the invention, added to the hyaluronic acid compositions, both in the form of injectable fillers and topical formulations, in percentages by weight on the total of the formulation comprised between 0.1 and 10 % by weight, slows down the degradation process of the HA, increasing its permanence in the application site and therefore the duration of action.

A further object of the invention therefore relates to the use of a mixture of glycine, alanine, valine, leucine, proline and lysine to counteract the degradation of injectable (e.g. filler or bio-revitalizing) or topical compositions based on hyaluronic acid. For this purpose, the additive may be added to the composition or may also be administered separately at the site of application of the filler or biorivitalizer or topical composition.

The invention finally relates to a method for inhibiting the degradation of hyaluronic acid injected or topically applied for cosmetic purposes which comprises the simultaneous, separate or sequential administration of a mixture of glycine, alanine, valine, leucine, proline and lysine.

The anti-hyaluronidase activity of the additive of the invention has been highlighted by turbidimetric method which offers the advantage of being fast and simple, therefore suitable for mass analysis. This method was initially described by Kass and Seastone (1944) [14] and later modified (Leonard, Perlman and Kurzrok, 1944; Meyer, 1947; Dorfman and Ott, 1948 [15]; Warren, Durso and Levin, 1948 [16]). The principle of the method is based on the fact that pure HA gives a relatively stable colloidal suspension with acidified diluted serum; the turbidity obtained is proportional to the concentration of HA. If the HA has been depolymerized with hyaluronidase, the turbidity is reduced.

One unit of hyaluronidase is defined as the quantity of enzyme capable, at 37°C and in 30 minutes, of reducing the turbidity provided by 0.2 mg of HA to that provided by 0.1 mg of HA. The effect of the enzyme depends on the salt concentration and the pH.

Thus, the assay for hyaluronidase (EC 3.2.1.35, HAse) determines activity indirectly by measuring the amount of undegraded HA substrate remaining after the enzyme has been allowed to react with the HA for 45 minutes at 37°C.

Hyaluronidase activity was assayed in solutions of HA (0.3 mg/ml) or HA plus amino acids (AA, HA + lysine, HA + glycine and proline, HA + lysine, glycine and proline) to consider two mixtures of HA in combination with six amino acids (glycine, proline, leucine, lysine, valine, alanine), with the second association with an increased amino acid content (+ 28%). The mixtures were incubated with the enzyme solution (hyaluronidase, Sigma-Aldrich) in enzyme diluent (20 mM sodium phosphate with 77 mM sodium chloride and 0.01% bovine serum albumin) for 45 min at 37°C. At the end, the acidic albumin solution (24 mM sodium acetate, 79 mM acetic acid with 0.1% bovine serum albumin) was added for 10 minutes at room temperature, then the absorbance was read at 600 nm.

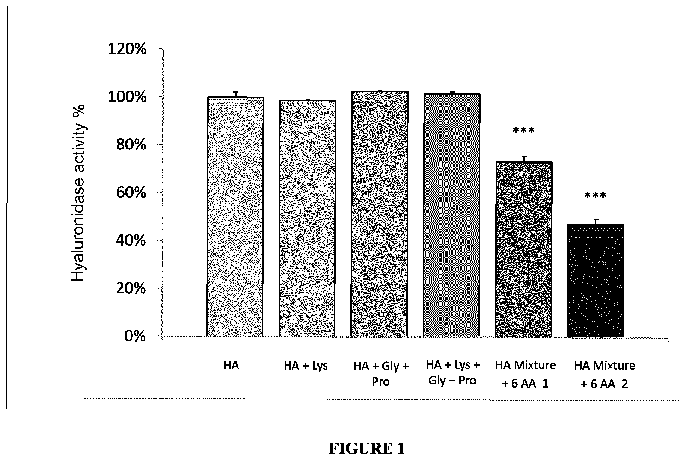

Figure 1 shows the hyaluronidase activity of HA or HA plus AA solutions measured using the turbidimetric assay. The results are means ± SD of duplicates of three independent experiments. *** P value < 0.005 compared to HA solution. The results show that only mixtures with six AAs are able to decrease the activity of hyaluronidase. In particular, the product containing 0.302 mg/mL glycine, 0.227 mg/mL proline, 0.042 mg/mL leucine, 0.033 mg/mL lysine, 0.168 mg/mL valine and 0.228 m/mL alanine (mixture 1) reduced the enzymatic activity by 27%, while the second mixture, with an increase in AA concentrations by 28%, resulted in a reduction of 53% compared to the HA solution alone (without the AA mixture). The response therefore appears dose-dependent.

The results demonstrate that the mixture of amino acids according to the invention allows a significant reduction of the degradation process of HA which can therefore persist longer and be more effective than HA formulated without amino acids.

Other tests were also carried out with a different approach in order to evaluate in a biochemical-instrumental system the susceptibility, and consequently the accessibility, of an injectable product containing hyaluronic acid and amino acids, which are able to modify the environment and the activity of the hyaluronidase enzyme. The enzymatic activity of hyaluronidases was monitored by spectrophotometric readings, according to the procedure described by Tolksdorf et al [17] and Kass and Seatone [14], based on the ability of hyaluronic acid to produce turbidity in the presence of a standard solution of albumin; turbidity is a function of the concentration of hyaluronic acid. Experimental comparisons were carried out with respect to a solution of hyaluronic acid, tested at the same concentrations and conditions of the test product, prepared starting from sodium hyaluronate without amino acids. The experimental protocol took into account the change in the absorbance of the hyaluronic acid solution and the pH over time.

Initially, the injectable form of the mixture of HA and additives with the following composition was considered: water for injection, sodium hyaluronate, glycine, L-proline, L-leucine, L-lysine, L-valine, L-alanine. The sample of hyaluronic acid without amino acids (50-250 kDa) used for the experimental comparisons was prepared starting from the hyaluronate sodium powder (35 mg in 3.5mL. The hyaluronate solution without amino acids was prepared at 1%. The test sample was added as such into the reaction mix.

For the evaluation of the activity of the hyaluronidase enzyme, the following were prepared in a test tube: - 0.1 M sodium phosphate buffer, pH 5.3 with 0.15 M NaCl (HSE buffer) - 0.5 M sodium acetate buffer, pH 4.2 - albumin (2.5 g serum bovine albumin in 1000 ml in sodium acetate buffer) - Hyaluronidase 0.01 mg/ml in 0.1 M HSE buffer. The samples to be tested were added to the reaction mix (each product was tested separately). The turbidity (OD) of the reaction mixture was then measured at T0, T30 (after 30 minutes), T60 (after 60 minutes) and T120 (after 120 minutes). The data were processed to evaluate the activity of the hyaluronidase enzyme on the test sample. The tests were performed in triplicate and the reported data refer to the average of the data obtained in the individual tests for each monitored time. In addition, pH measurement was performed at each experimental time to assess the influence of the amino acids on the electrical charges of the system (sample + reaction mix). In parallel, all evaluations were also performed on the reference sample of hyaluronic acid without amino acids.

The results obtained reported as a percentage of hydrolysed hyaluronic acid (expressed as a reduction in absorbance over time) for each time analysed, calculated as OD VAR% vs T0, are reported in Table 1.

**Table 1**

| **HA+ Glycine, L-Proline, L-Leucine, L-Lysine, L-Valine, L-Alanine mixture** | **HYALURONIDASE ACTIVITY measured as VAR % OD vs T0 *measured as OD VAR* % *vs T0*** |
|---|---|
| T0 VS T30 | 1.2% |
| T0 VS T60 | 4.7% |
| T0 VS T120 | 5.4% |

The results are reported as a percentage of hydrolysed hyaluronic acid (expressed as a reduction in absorbance over time) for each time analysed, calculated as OD VAR% vs T0.

The results obtained for the sample of hyaluronic acid without amino acid used for the comparisons are reported in Table 2. The results are reported as a percentage of hydrolysed hyaluronic acid (expressed as a reduction in absorbance over time) for each time analysed, calculated as OD VAR% vs T0.

**Table 2**

| **HYALURONIC ACID (50-250 kDa)** | **HYALURONIDASE ACTIVITY/ measured as VAR % OD vs T0 *measured as OD VAR* % *vs T0*** |
|---|---|
| **Without amino acids** | |
| T0 VS T30 | 4.9% |
| T0 VS T60 | 11.6% |
| T0 VS T120 | 20.6% |

The results relating to the pH value of the solution containing the test sample of hyaluronic acid with and without amino acids and the reaction mixture with hyaluronidase at each time analysed are reported in Table 3.

**Table 3**

| **Experimental times** | **HA+ Glycine, L-Proline, L-Leucine, L-Lysine, L-Valine, L-Alanine mixture** | **HYALURONIC ACID (50-250 kDa)** |
|---|---|---|
| | | **Without amino acids** |
| T0 | 7.182 | 7.286 |
| T30 | 7.031 | 7.159 |
| T60 | 7.030 | 7.096 |
| T120 | 7.001 | 6.960 |

With the same method, a composition in the form of a cream was evaluated, which consisted as follows: purified water, sodium hyaluronate, glycine, L-proline, L-leucine, L-lysine, HCl, L-valine, L-alanine, cetylstearyl octanoate, copolymer of acrylic acid and vinyl ester, cetyl stearyl alcohol, potassium cetyl phosphate, imidazolidinyl urea, Faracide ME, sodium hydroxide. The amino acid-free hyaluronic acid sample used for the experimental comparisons was prepared starting from the hyaluronate sodium powder with molecular weight 1800-2200 kDa. Considering that the hyaluronic acid present in the formulation is 2 g per 100 g of product, 2% sodium hyaluronate without amino acids was used.
The results obtained reported as a percentage of hydrolysed hyaluronic acid (expressed as a reduction in absorbance over time) for each time analysed, calculated as OD VAR% vs T0, are reported in Table 4.

**Table 4**

| **HA +AA cream formulation** | **HYALURONIDASE ACTIVITY/ measured as VAR % OD vs T0 *measured as OD VAR* % *vs T0*** |
|---|---|
| T0 VS T30 | 4.0% |
| T0 VS T60 | 4.3% |
| T0 VS T120 | 5.5% |

The results are reported as a percentage of hydrolysed hyaluronic acid (expressed as a reduction in absorbance over time) for each time analysed, calculated as OD VAR% vs T0.

The results obtained for the sample of hyaluronic acid without amino acid used for the comparisons are reported in Table 5. The results are reported as a percentage of hydrolysed hyaluronic acid (expressed as a reduction in absorbance over time) for each time analysed, calculated as OD VAR% vs T0.

**Table 5**

| **HYALURONIC ACID (1800-2200 kDa)** | **HYALURONIDASE ACTIVITY/ measured as VAR % OD vs T0 *measured as OD VAR* % *vs T0*** |
|---|---|
| **Without amino acids** | |
| T0 VS T30 | 12.7% |
| T0 VS T60 | 23.5% |
| T0 VS T120 | 39.7% |

The results relating to the pH value of the solution containing the test sample of hyaluronic acid with and without amino acids and the reaction mixture with hyaluronidase at each time analysed are reported in Table 6.

**Table 6**

| **.Experimental times** | **pH measurement** | **HYALURONIC ACID (1800-2200 kDa)** |
|---|---|---|
| | | **Without amino acids** |
| T0 | 6.340 | 7.350 |
| T30 | 6.310 | 7.298 |
| T60 | 6.256 | 7.033 |
| T120 | 6.276 | 6.933 |

Finally, again with the same method, a gel composition was evaluated, which consisted as follows: purified water, sodium hyaluronate, glycine, L-proline, L-leucine, L-lysine, HCl, L-valine, L-alanine, methyl paraoxybenzoate, propyl paraoxybenzoate, Propylene Glycol. The amino acid-free hyaluronic acid sample used for the experimental comparisons was prepared starting from the hyaluronate sodium powder with molecular weight 1800-2200 kDa.
Considering that the hyaluronic acid present in the formulation is 2 g per 100 g of product, 2% sodium hyaluronate without amino acids was used.
The results obtained reported as a percentage of hydrolysed hyaluronic acid (expressed as a reduction in absorbance over time) for each time analysed, calculated as OD VAR% vs T0, are reported in Table 7.

| **HA+AA gel formulation** | **HYALURONIDASE ACTIVITY/ measured as VAR % OD vs T0 *measured as OD VAR* % *vs T0*** |
|---|---|
| T0 VS T30 | 10.8% |
| T0 VS T60 | 12.3% |
| T0 VS T120 | 19.6% |

The results are reported as a percentage of hydrolysed hyaluronic acid (expressed as a reduction in absorbance over time) for each time analysed, calculated as OD VAR% vs T0.
The results obtained for the sample of hyaluronic acid without amino acid used for the comparisons are reported in Table 8. The results are reported as a percentage of hydrolysed hyaluronic acid (expressed as a reduction in absorbance over time) for each time analysed, calculated as OD VAR% vs T0.

**Table 8**

| **HYALURONIC ACID (1800-2200 kDa)** | **HYALURONIDASE ACTIVITY/ measured as VAR % OD vs T0 *measured as OD VAR* % *vs T0*** |
|---|---|
| **Without amino acids** | |
| T0 VS T30 | 12.7% |
| T0 VS T60 | 23.5% |
| T0 VS T120 | 39.7% |

The results relating to the pH value of the solution containing the test sample of hyaluronic acid with and without amino acids and the reaction mixture with hyaluronidase at each time analysed are reported in Table 9.

**Table 9**

| **Experimental times** | **pH measurement** | **HYALURONIC ACID (1800-2200 kDa)** |
|---|---|---|
| | | **Without amino acids** |
| T0 | 6.934 | 7.350 |
| T30 | 6.822 | 7.298 |
| T60 | 6.710 | 7.033 |
| T120 | 6.688 | 6.933 |

The data of the above tests demonstrate that the mixture of amino acids, according to the invention, even with different forms of preparation, allows a reduction in the activity of hyaluronidase, with a stabilization of the acidity levels of the environment in which the enzyme operates. It follows that HA in association with amino acids may therefore persist longer and be more effective than HA formulated without them.

### References

[1] G. Kreil, Protein Sci. 4 (1995) 1666-1669.
[2] T.B. Csóka et al., Invasion Metastasis 17 (1997) 297-311.
[3] L.H. Chen et al., J Int. J. Biol. Macromol. 116 (2018) 572-584.
[4] Z. Hussain, et al., Polym. Rev. 57 (2017) 594-630.
[5] B. Fiszer-Szafarz et al., Biol. Cell. 63 (1988) 355-360.
[6] M.H. Safdar et al., Artif. Cells Nanomed. Biotechnol. (2017) 1-14.
[7] N.I. Ievdokimova, Ukr. Biokhim. Zh. 80 (2008) 5-44.
[8] E. Papakonstantinou et al., Dermatoendocrinol. 4 (2012) 253-258.
[9] V.A. Narurkar et al., J. Drugs Dermatol. 15 (2016) S24-S37.
[10] T. Pavicic et al., J. Drugs Dermatol. 9 (2011) 990-1000.
[11] Payne WM et al., Carbohydr Polym. 2018 Feb 15; 182:132-141.
[12] Tiwari S et al., Int J Biol Macromol. 2019 Jan; 121:556-571.
[13] Labie H et al., J Colloid Interface Sci. 2019 Feb 1;535:16-27.
[14] E H Kass et al., J Exp Med. 1944 Mar 1;79(3):319-30.
[15] A Dorfman, M L Ott. J Biol Chem. 1948 Feb;172(2):367-75.
[16] G H Warren et al., Endocrinologia. 1948 Jul;43(1):48-51.
[17] Tolksdorf, S., et al., J Lab Clin Med 34, 74, 1949

## Claims

1. An additive with anti-hyaluronidase activity comprising a mixture of glycine, alanine, valine, leucine, proline and lysine.

2. Use of a mixture of glycine, alanine, valine, leucine, proline and lysine to counteract the degradation of injective compositions such as fillers or bio-revitalizers or of topical compositions based on hyaluronic acid.

3. Additive according to claim 2, wherein the amino acids are of the L series.

4. A method for inhibiting the degradation of hyaluronic acid injected or applied topically for cosmetic purposes which comprises the simultaneous, separate or sequential administration of a mixture of glycine, alanine, valine, leucine, proline and lysine.
